# EUROPÄISCHE PATENTSCHRIFT

(11) **EP 0 508 095 B1**
(45) Veröffentlichungstag und Bekanntmachung des Hinweises auf die Patenterteilung: **18.06.1997**
(21) Anmeldenummer: 92103632.3
(22) Anmeldetag: 03.03.1992
(51) Int. Cl.: A61K 6/083, A61K 6/02

(54) **Dentalwerkstoffe auf Basis von (Meth)-Acrylaten**
Dental materials based on (meth)-acrylates
Matériaux dentaires basés sur des (meth)-acrylates

(30) Priorität: 16.03.1991 DE 4108634
(43) Veröffentlichungstag der Anmeldung: 14.10.1992
(73) Patentinhaber: Heraeus Kulzer GmbH & Co.KG, 63450 Hanau (DE)
(72) Erfinder: Podszun, Wolfgang, Dr., W-5000 Köln 80 (DE); Winkel, Jens, Dr., W-5000 Köln (DE); Schwabe, Peter, Dr., W-5090 Leverkusen 1 (DE)
(74) Vertreter: Kühn, Hans-Christian

(56) Entgegenhaltungen:
- EP-A- 0 011 734
- EP-A- 0 059 525
- EP-A- 0 166 009
- EP-A- 0 346 707
- EP-A- 0 427 300
- DE-B- 2 403 211

## Beschreibung

Die Erfindung betrifft Dentalwerkstoffe, ihre Herstellung und ihre Verwendung.

Dentalwerkstoffe können beispielsweise zur Herstellung von künstlichen Zähnen, Kronen, Brücken, Inlays, Onlays, Zahnfüllungen und Zahnlacken eingesetzt werden.

Es ist bekannt, Dentalwerkstoffe auf Basis von polymeren (Meth)-Acrylaten herzustellen. So stellt man beispielsweise Werkstoffe her, die Polymethylmethacrylat-Perlpolymerisate als Pulverkomponente und Mischungen aus Methylmethacrylat und Ethylenglykoldimethacrylat enthalten; die Mischungen härten im allgemeinen durch radikalische Polymerisation unter Formgebung aus (Ullmann's Encyclopedia of Industrial Chemistry, Fifth Edition, Volume A8, S. 277 ff., VCH Verlagsgesellschaft m.b.H., Weinheim 1987).

In US 4 396 377 werden Dentalmaterialien zur Herstellung von Kunststoffzähnen vorgeschlagen, die als Pulver (neben unvernetztem) vernetztes Polymethylmethacrylat enthalten. Als Monomerflüssigkeit wird das herkömmliche Gemisch aus Methylmethacrylat und einem Vernetzer wie Ethylenglykoldimethacrylat verwendet. Dentalmaterialien gemäß US 4 396 377 weisen die folgende Zusammensetzung auf:
- 0-50 %: unvernetztes Polymer
- 10-70 %: vernetztes Polymer
- 20-66 %: Monomer (welches nicht als Vernetzer wirkt)
- 7-27 %: Vernetzer.

EP-A 59 525 beschreibt ähnliche Materialien wie die vorstehend zitierte Patentschrift. Die beanspruchten Materialien haben die folgende Zusammensetzung:
- 0-50 %: unvernetztes Polymer
- 10-70 %: vernetztes Polymer
- 2-30 %: Monomer (welches nicht als Vernetzer wirkt)
- 20-70 %: Vernetzer.

Aus EP-A 346 707 sind Dentalwerkstoffe zur Herstellung von künstlichen Zähnen bekannt geworden, bei denen als Monomerflüssigkeit ausschließlich oder überwiegend Vernetzer und als Polymerkomponente ausschließlich vernetztes Polymerisat mit besonderen Quellungseigenschaften Verwendung findet. Diese Dentalwerkstoffe haben die nachfolgende Zusammensetzung:
- 5-35 %: vernetztes Polymer
- 0-40 %: Monomer (welches nicht als Vernetzer wirkt)
- 40-90 %: Vernetzer.

Aus EP-A-0 166 009 sind Dentalwerkstoffe bekannt geworden, bei denen nur geringe Mengen in Vernetzern eingesetzt werden. Vernetzungsgrade von 50-100 % lassen sich damit nicht erzielen.

DE-A 2 403 211 beschreibt füllstoffhaltige Dentalmassen, die dadurch gekennzeichnet sind, daß sie als Füllstoff ausschließlich mikrofeines Siliziumdioxid und als Monomer Bis-GMA oder spezielle durch Umsetzung von Diisocyanaten mit Hydroxyalkylmethacrylaten erhaltene Urethanmethacrylate enthalten.

Es wurden Dentalwerkstoffe aus
a) 5 bis 30 Gew.-Teilen eines organischen Füllstoffs, bestehend aus polymeren vernetzten (Meth)-Acrylaten mit einer Teilchengröße im Bereich von 0,001 bis 100 µm, einem Quellungsgrad von 50 bis 2000 Gew.-% und einem Vernetzungsgrad von 50 bis 100 Gew.-%, jeweils bezogen auf das Polymer,
b) 40 bis 80 Gew.-Teilen (Meth)-Acrylate, die Vernetzungen ausbilden können,
c) 0 bis 40 Gew.-Teilen (Meth)-Acrylate, die keine Vernetzungen ausbilden können,
d) 10 bis 40 Gew.-Teilen eines silanisierten anorganischen Füllstoffs mit einer mittleren Teilchengröße von 0,005 bis 5 µm und
e) 0,1 bis 10 Gew.-%, bezogen auf die Gesamtzusammensetzung, eines oder mehrerer Additive,
gefunden.

Die erfindungsgemäßen Dentalwerkstoffe zeichnen sich überraschenderweise durch eine besonders hohe Härte und Steifigkeit aus. Sie lassen sich zu sehr dünnen Schichten verarbeiten.

Sie lassen sich sehr gut polieren und ergeben dabei harte, hochglänzende Oberflächen. Die Verschleißfestigkeit der erfindungsgemäßen Dentalwerkstoffe ist sehr hoch. Nach Zusatz von Pigmenten wird eine hervorragende Angleichung an das Aussehen natürlicher Zähne erreicht.

### Komponente (a)

Organische Füllstoffe im Rahmen der Erfindung sind polymere vernetzte (Meth)-Acrylate mit einer Teilchengröße im Bereich von 0,001 bis 100 µm, vorzugsweise 0,01 bis 10 µm, die einen Quellungsgrad von 50 bis 2000 Gew.-% und einen Vernetzungsgrad von 50 bis 100 Gew.-%, jeweils bezogen auf das Polymer, aufweisen.

Als Vernetzungsgrad wird hier der Anteil an (Meth)-Acrylaten, die Vernetzungen, bezogen auf das Polymer ausbilden können, definiert.

Monomere (Meth)-Acrylate der organischen Füllstoffe, die Vernetzungen ausbilden, sind (Meth)-Acrylate mit 2 oder mehr, bevorzugt 2 bis 4, polymerisierbaren Doppelbindungen im Molekül.

Als monomere (Meth)-Acrylate, die Vernetzungen ausbilden, seien beispielsweise genannt:

Ethylenglykoldimethacrylat, Diethylenglykoldimethacrylat, Triethylenglykoldimethacrylat, Glycerindimethacrylat, Glycerintrimethacrylat, Trimethylolpropantrimethacrylat, Pentaerythritdimethacrylat, Pentaerythrittrimethacrylat, Pentaerythrittetramethacrylat, Derivate des Bisphenol A, wie Bisphenol-A-dimethacrylat und Bisphenol-A-diglycidyldimethacrylat, Urethanmethacrylate, die durch Umsetzung von Diisocyanaten und Hydroxyalkylmethacrylaten hergestellt werden können, wie

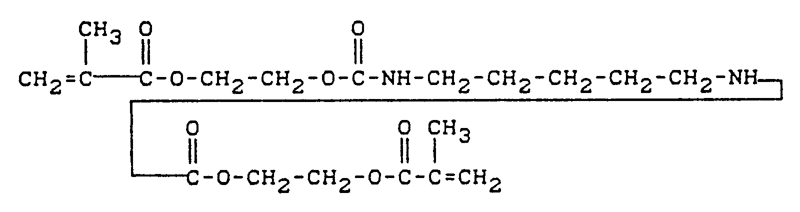

Umsetzungsprodukte aus Polyolen, Diisocyanaten und Hydroxyalkylmethacrylaten (DE-A 37 03 080, DE-A 37 03 130 und DE-A 37 03 120), wie z.B.

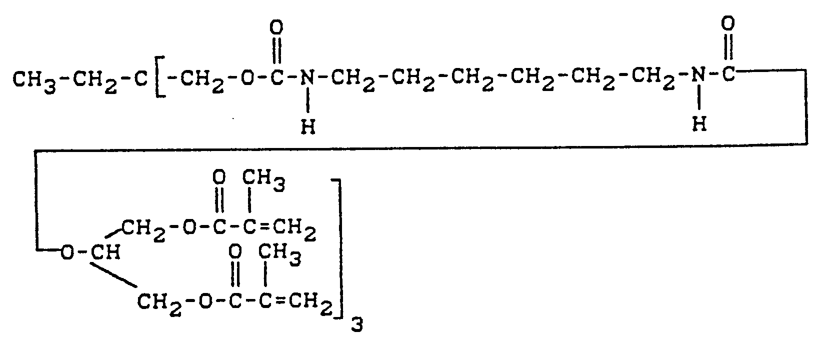

Bevorzugt werden monomere (Meth)-Acrylate, die Vernetzungen ausbilden, wie:

Ethylenglykoldimethacrylat, Diethylenglykoldimethacrylat, Triethylenglykoldimethacrylat und Glycerindimethacrylat.

Als monomere (Meth)-Acrylate der Füllstoffe, die keine Vernetzungen ausbilden, seien beispielsweise genannt:

C₁-C₁₂-, bevorzugt C₁-C₄-Alkylmethacrylate, wie Methylmethacrylat, Ethylmethacrylat, n-Propylmethacrylat, iso-Propylmethacrylat, n-Butylmethacrylat, t-Butylmethacrylat,
Hydroxyalkyl(C₁-C₄)methacrylate, wie 2-Hydroxyethylmethacrylat, 2-Hydroxypropylmethacrylat, Diethylenglykolmonomethacrylat, Triethylenglykolmonomethacrylat, Alkoxy(C₁-C₄)ethylmethacrylat, wie 2-Methoxyethylmethacrylat, 3-Methoxybutylmethacrylat und Ethyltriglykolmethacrylat.

Bevorzugte monomere (Meth)-Acrylate, die keine Vernetzungen ausbilden, sind beispielsweise:

Methylmethacrylat, Ethylmethacrylat, 2-Hydroxyethylmethacrylat.

Die monomeren (Meth)-Acrylate sind an sich bekannt und können beispielsweise durch Umsetzung von (Meth)-Acrylsäurechlorid mit den entsprechenden Alkoholen hergestellt werden.

Unter dem Quellungsgrad versteht man das Aufnahmevermögen der erfindungsgemäßen Poly(meth)acrylate an Flüssigkeit. Der Quellungsgrad wird gemessen durch das Aufnahmevermögen an Tetrahydrofuran bei 20°C. Die erfindungsgemäßen Poly(meth)acrylate weisen ein Quellungsvermögen von 50 bis 2000 Gew.-%, bevorzugt von 100 bis 1000 Gew.-%, bezogen auf das Polymer, auf.

Die erfindungsgemäßen organischen Füllstoffe weisen im allgemeinen einen mittleren Teilchendurchmesser von 0,001 bis 100 µm, bevorzugt von 0,01 bis 10 µm, auf.

Bevorzugt weisen die erfindungsgemäßen organischen Füllstoffe einen Gelgehalt von 5 bis 100 Gew.-%, bevorzugt von 95 bis 100 Gew.-%, bezogen auf das Polymer, auf. Im Rahmen der vorliegenden Erfindung versteht man unter dem Gelgehalt definitionsgemäß den Anteil des Polymers, der mit Tetrahydrofuran als Lösungsmittel bei 20° C nicht löslich ist. Der Gelgehalt ist eine Kenngröße für die tatsächlich eingetretene Vernetzung.

Die erfindungsgemäßen organischen Füllstoffe weisen eine aktive Oberfläche von 10 bis 600 m²/g, bevorzugt von 50 bis 300 m²/g, gemessen nach der BET-Methode, auf.

Erfindungsgemäße organische Füllstoffe mit einer Teilchengröße von ca. 5 - 100 µm lassen sich nach dem Verfahren der Suspensionspolymerisation synthetisieren.

Bevorzugte erfindungsgemäße organische Füllstoffe mit einer Teilchengröße von 0,001 bis 10 µm lassen sich dadurch herstellen, daß man (Meth)-Acrylate, die Vernetzungen ausbilden, und gegebenenfalls (Meth)-Acrylate, die keine Vernetzungen ausbilden, in Gegenwart eines organischen Lösungsmittels mit einem Löslichkeitsparameter von 8 bis 15 [cal^{0,5}cm^{-1,5}] polymerisiert, wobei der Anteil der (Meth)-Acrylate, die Vernetzungen ausbilden, 50 bis 100 Gew.-% beträgt.

Organische Lösungsmittel können durch den sogenannten Löslichkeitsparameter definiert werden (H.G. Elias, Makromoleküle, S. 192-196 (1981)). Für das erfindungsgemäße Verfahren verwendet man Lösungsmittel mit einem Parameter von 8 bis 15 [cal^{0,5}cm^{-1,5}], bevorzugt von 8,5 bis 12 [cal^{0,5}cm^{-1,5}].

Beispielsweise seien die folgenden Lösungsmittel genannt:

Amylacetat, Tetrachlorethan, Toluol, Ethylacetat, Tetrahydrofuran, Benzol, Trichlormethan, Dichlormethan, Methylchlorid, Aceton, Butanon-2 und tert.-Butanol.

Die Menge des Lösungsmittels im Verhältnis zu den monomeren (Meth)-Acrylaten liegt im Bereich von 1:1 bis 1:100, vorzugsweise 1:2 bis 1:20.

Die Durchführung des Polymerisationsverfahrens erfolgt im allgemeinen im Temperaturbereich von 50 bis 250°C, bevorzugt von 60 bis 150°C. Dabei kann die Polymerisation kontinuierlich oder diskontinuierlich durchgeführt werden.

Die Polymerisation wird im allgemeinen in Gegenwart von Initiatoren wie Sensibilisatoren oder Radikalbildnern durchgeführt.

Die Initiatoren werden im allgemeinen in einer Menge von 0,01 bis 3 Gew.-%, vorzugsweise 0,1 bis 1,5 Gew.-%, bezogen auf das Gesamtmonomer, eingesetzt.

Als Polymerisationsinitiatoren können beispielsweise Perverbindungen oder Radikale liefernde Azoverbindungen verwendet werden. Beispielsweise seien genannt:

Aliphatische Azodicarbonsäurederivate wie Azoisobuttersäurenitril oder Azodicarbonsäureester, Peroxide wie Lauroylperoxid, Succinylperoxid, Dibenzoylperoxid, p-Chlorbenzoylperoxid, 2,4-Dichlorbenzoylperoxid, Peroxide wie Methylethylketonperoxid, Methylisobutylketonperoxid, Cyclohexanonperoxid, Acetylacetonperoxid, Alkylester von Persäuren wie tert.-Butylperpivalat, tert.-Butylperoctoat, tert.-Butylperbenzoat, tert.-Butylperisononat, Monotert.-butylpermaleinat, tert.-Butylperacetat, Percarbonate wie Dicyclohexyl- und Diisopropylpercarbonat, Dialkylperoxide wie Di-tert.-Butylperoxid, Dicumylperoxid, Hydroperoxide wie tert.-Butyl- oder Cumolhydroperoxid, Isophthalmonopersäure oder Acetylcyclohexansulfonylperoxid.

Bei der Polymerisation entsteht im allgemeinen eine Suspension des Füllstoffs. Die Isolierung des Füllstoffs kann beispielsweise durch Verdampfen des Lösungsmittels, beispielsweise in einem Sprühtrocknungsprozeß, erfolgen.

### Komponente (b)

(Meth)-Acrylsäureester, die Vernetzungen ausbilden können, enthalten im allgemeinen 2 oder mehr polymerisierbare aktive Gruppen, z.B. Doppelbindungen oder Isocyanatgruppen, im Molekül. Bevorzugt seien Ester der (Meth)-Acrylsäure mit 2- bis 5-wertigen Alkoholen mit 2 bis 30 Kohlenstoffatomen genannt. Besonders bevorzugt werden Epoxidmethacrylate und Urethanmethacrylate.

Beispielsweise seien (Meth)-Acrylsäureester der Formel 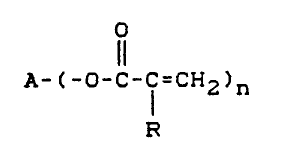 in der
- A: einen geradkettigen, verzweigten, cyclischen, aliphatischen, aromatischen oder gemischt aliphatisch-aromatischen Rest mit 2 bis 25 C-Atomen, der durch -O- oder NH-Brücken unterbrochen und mit Hydroxy, Oxy, Carboxy, Amino oder Halogen substituiert sein kann,
bedeutet,
- R: H oder Methyl bedeutet und
- n: für eine ganze Zahl von 2 bis 8, vorzugsweise 2 bis 4, steht,
genannt.

Vorzugsweise seien Verbindungen der folgenden Formeln genannt: 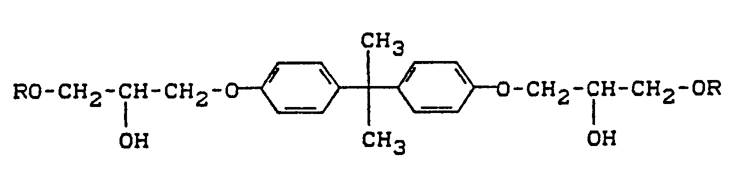 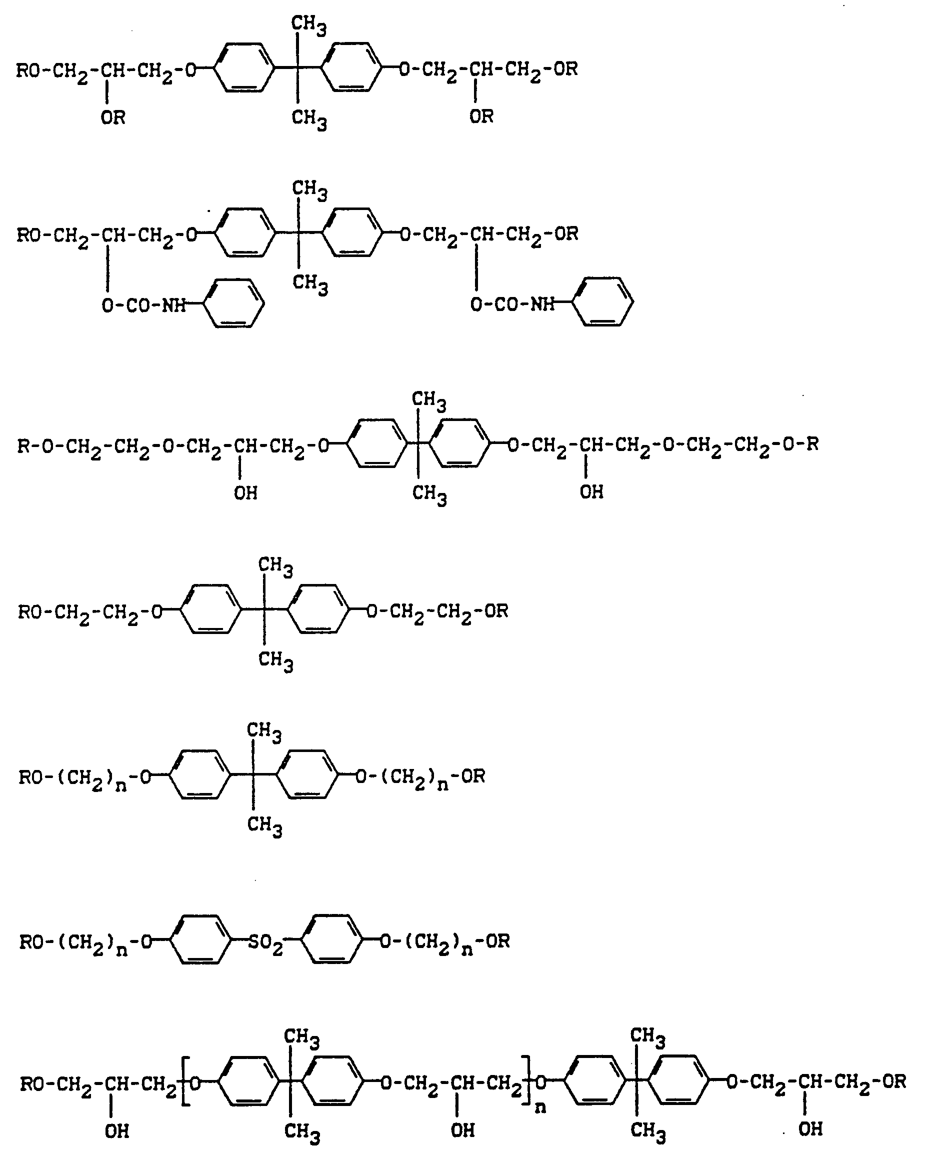 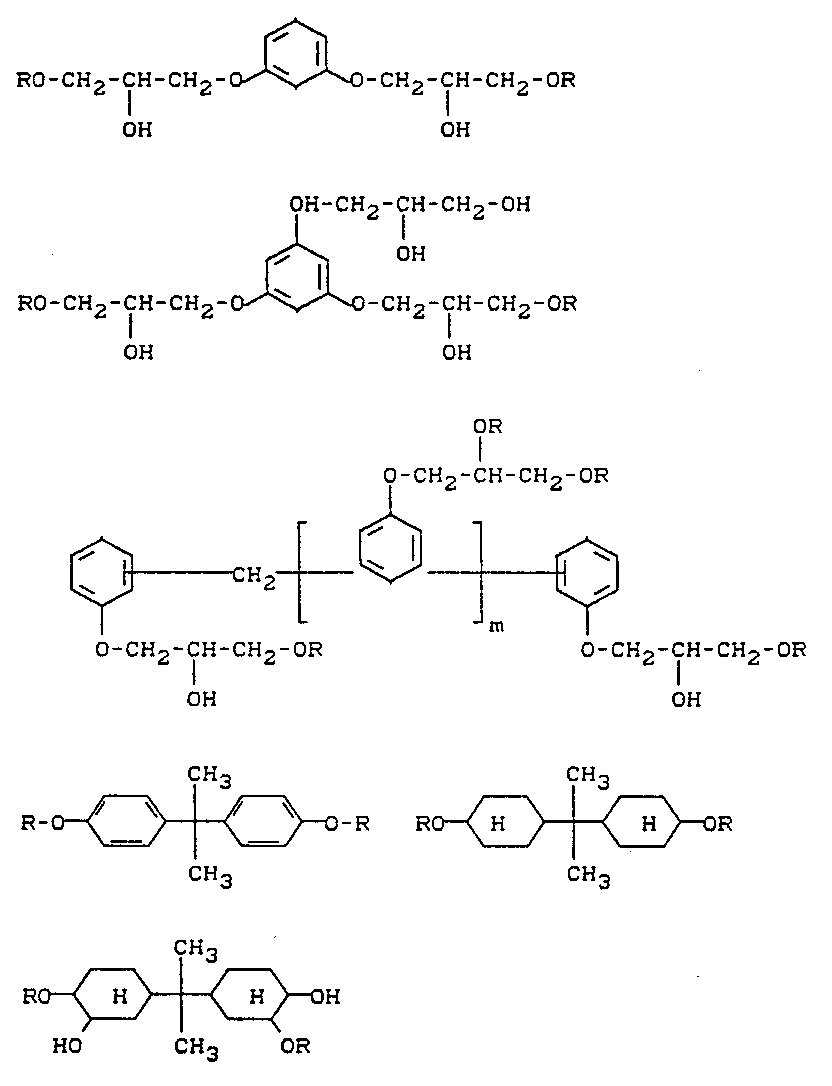 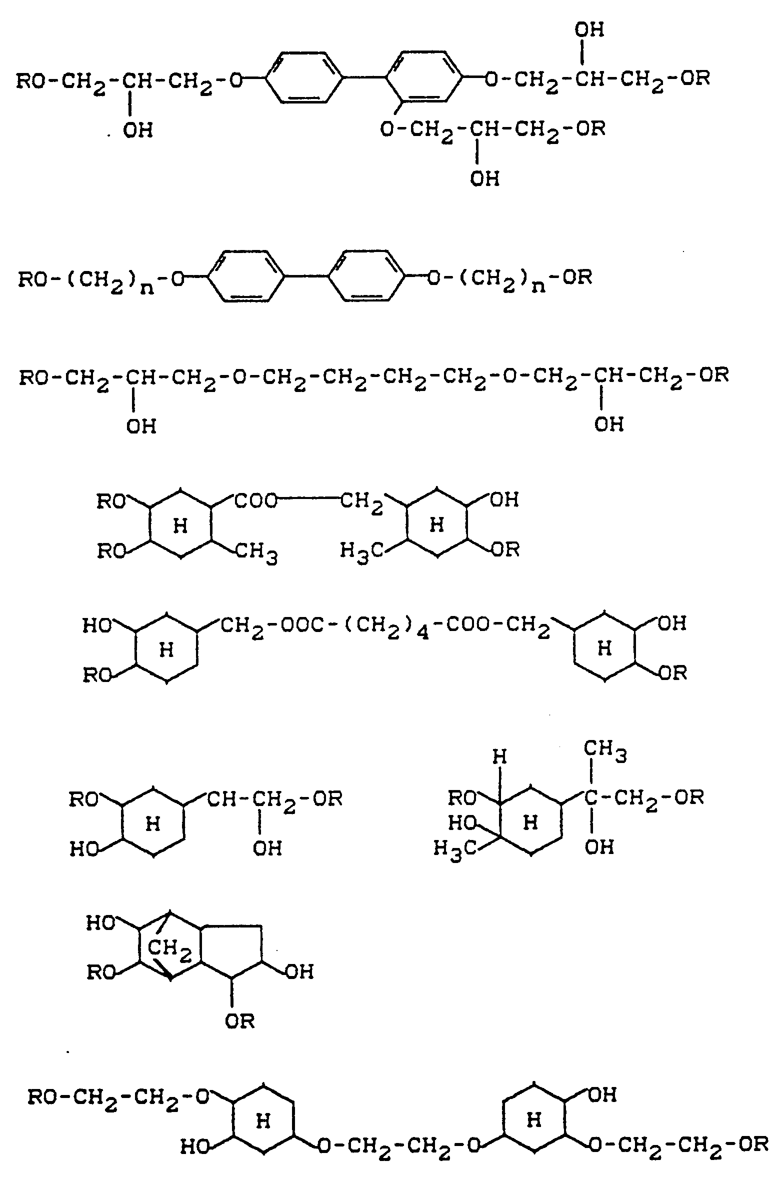 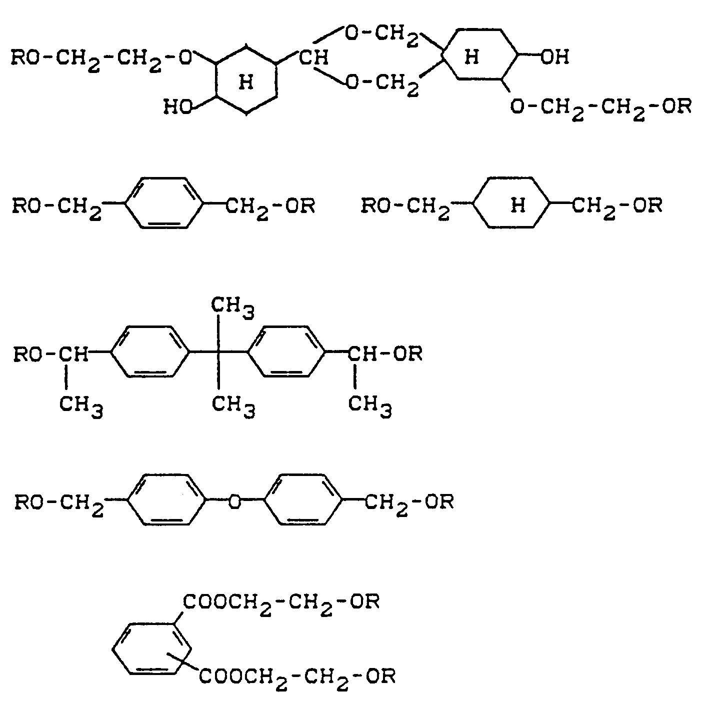 in der ortho-, meta- oder para-Form 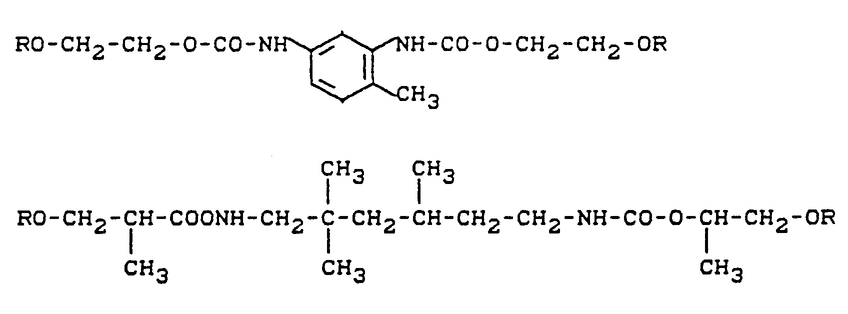 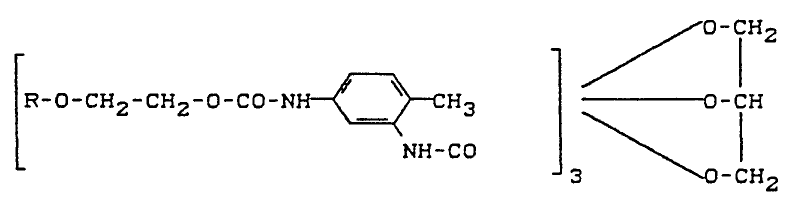 worin
- R: für 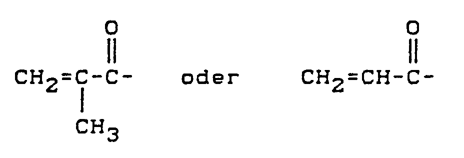 steht,
- n: eine Zahl von 1 bis 4 und
- m: eine Zahl von 0 bis 5 bedeutet.

Außerdem seien Derivate des Tricyclodecans (EP-A 00 23 686) und Umsetzungsprodukte aus Polyolen, Diisocyanaten und Hydroxyalkylmethacrylaten (DE-A 37 03 120, DE-A 37 03 080 und DE-A 37 03 130) genannt. Beispielsweise seien die folgenden Monomeren genannt:

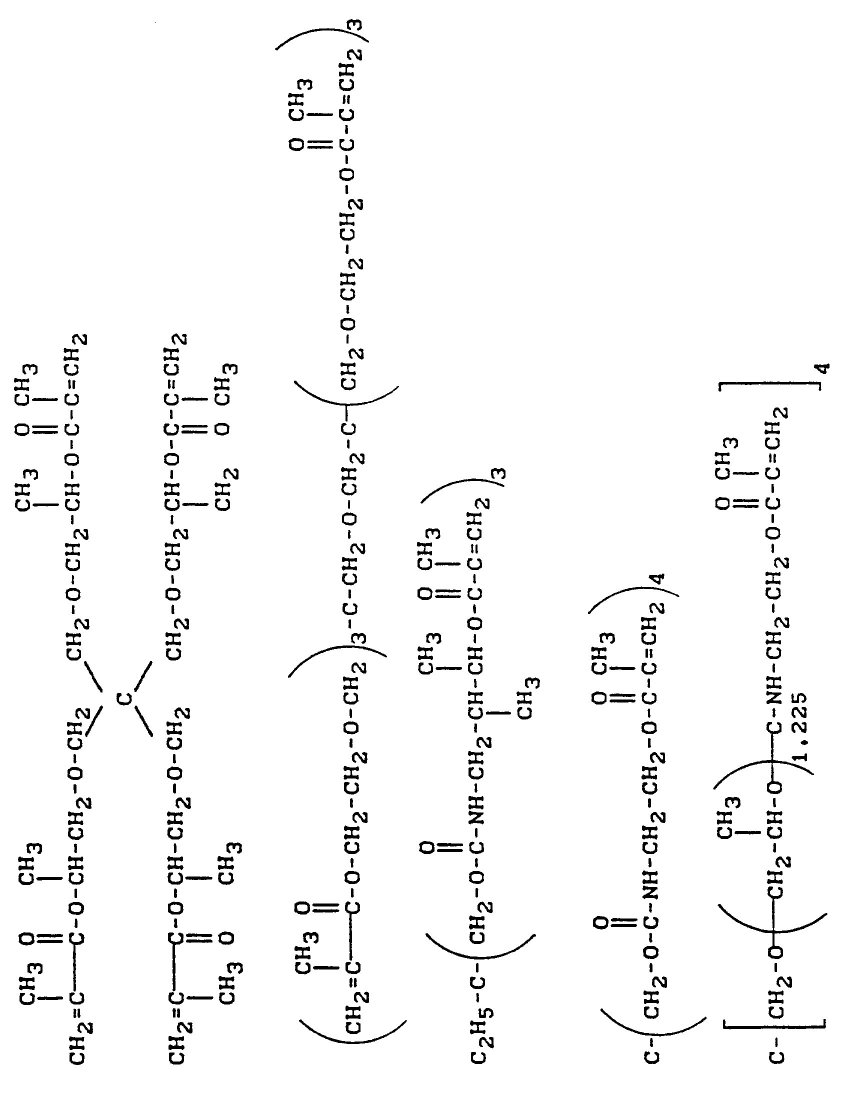

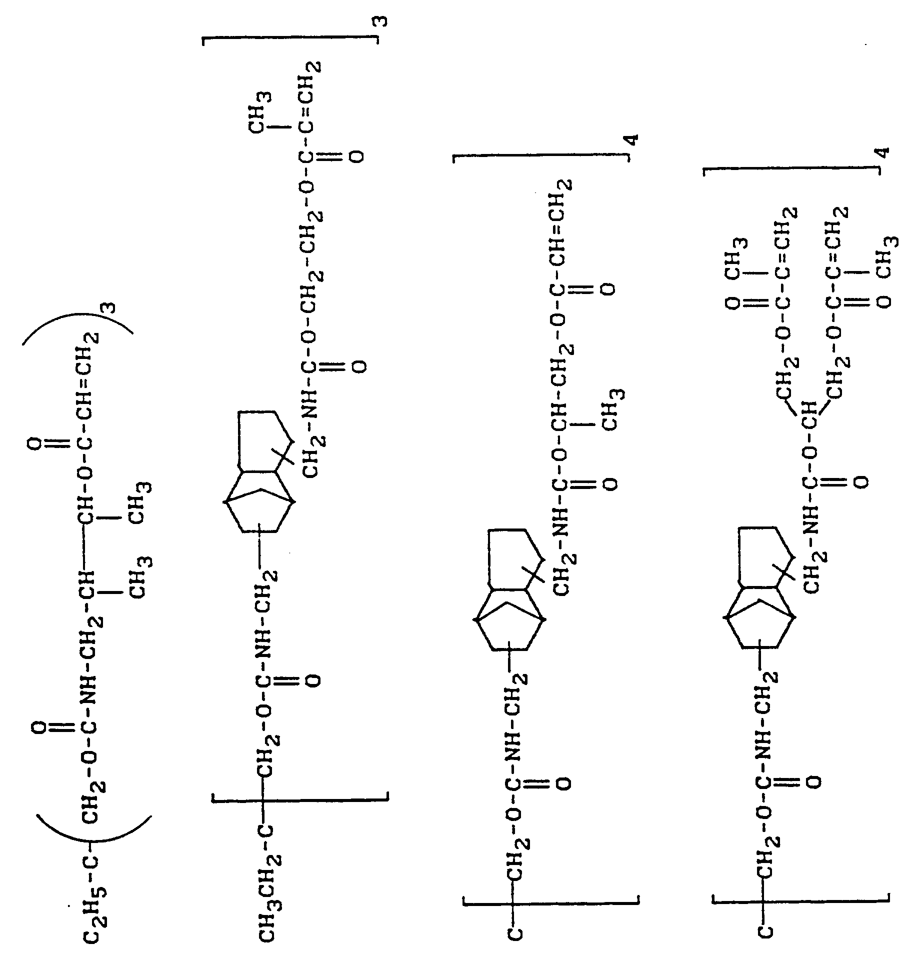

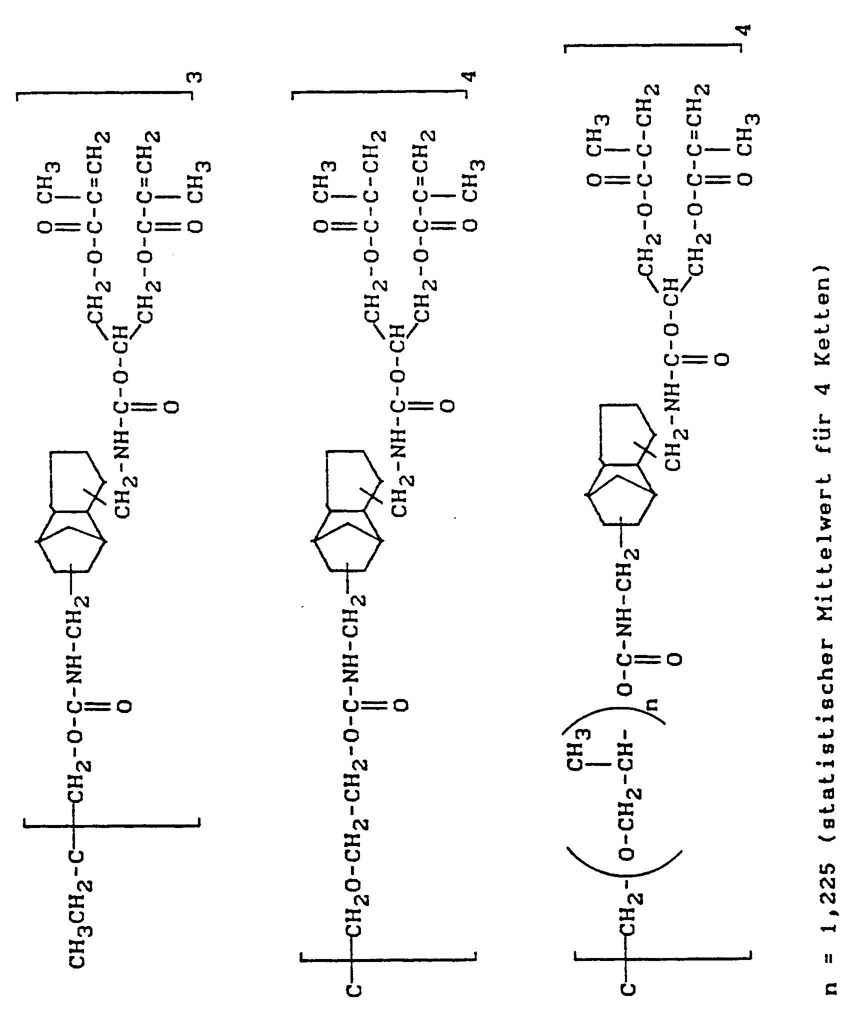

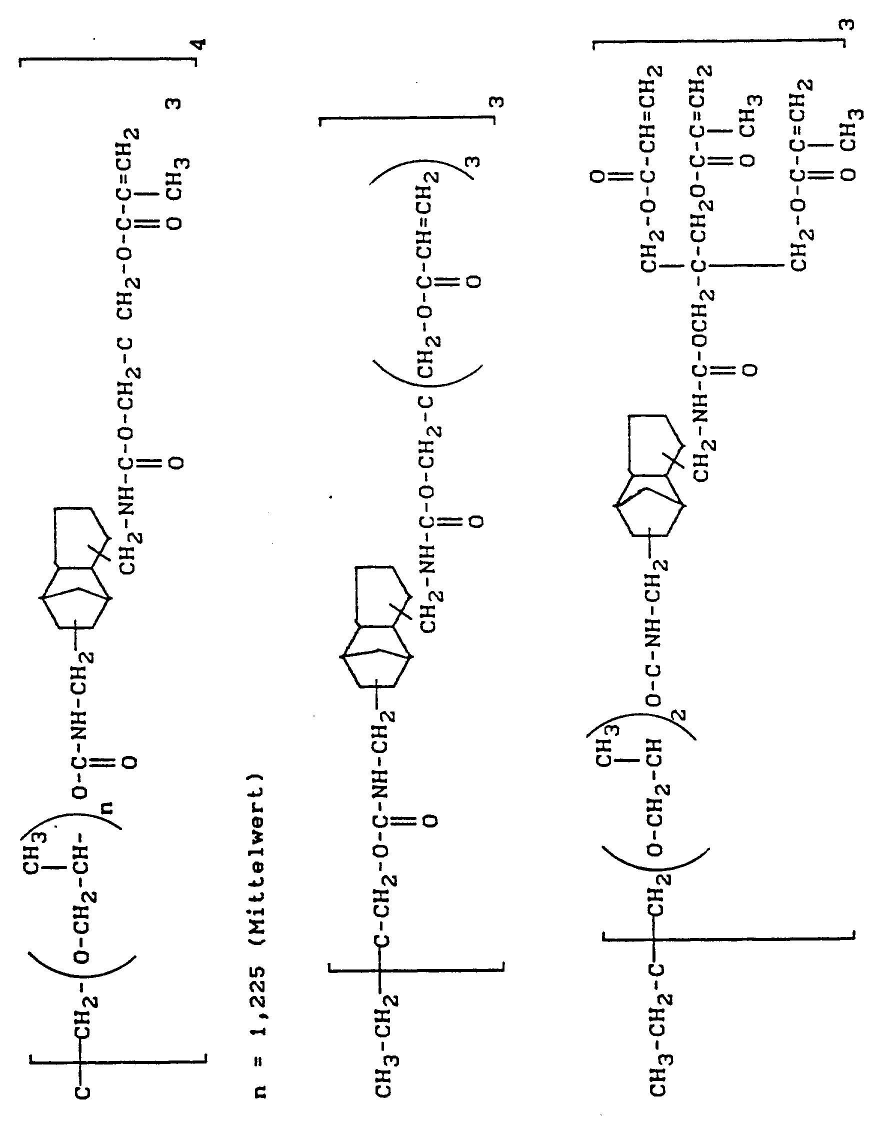

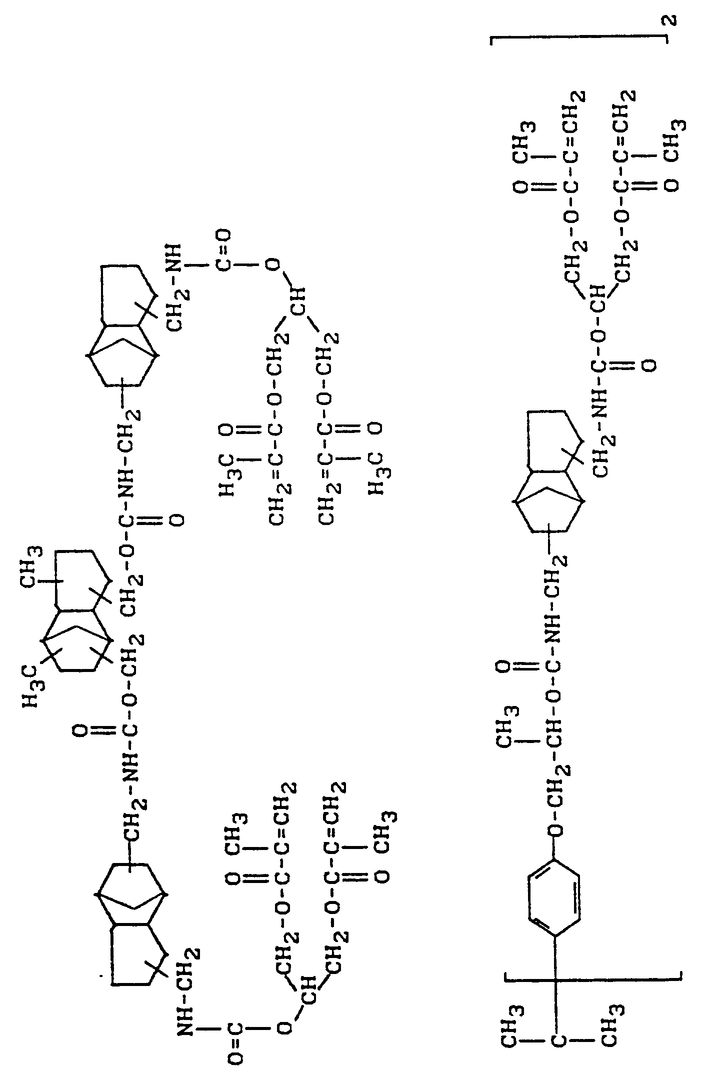

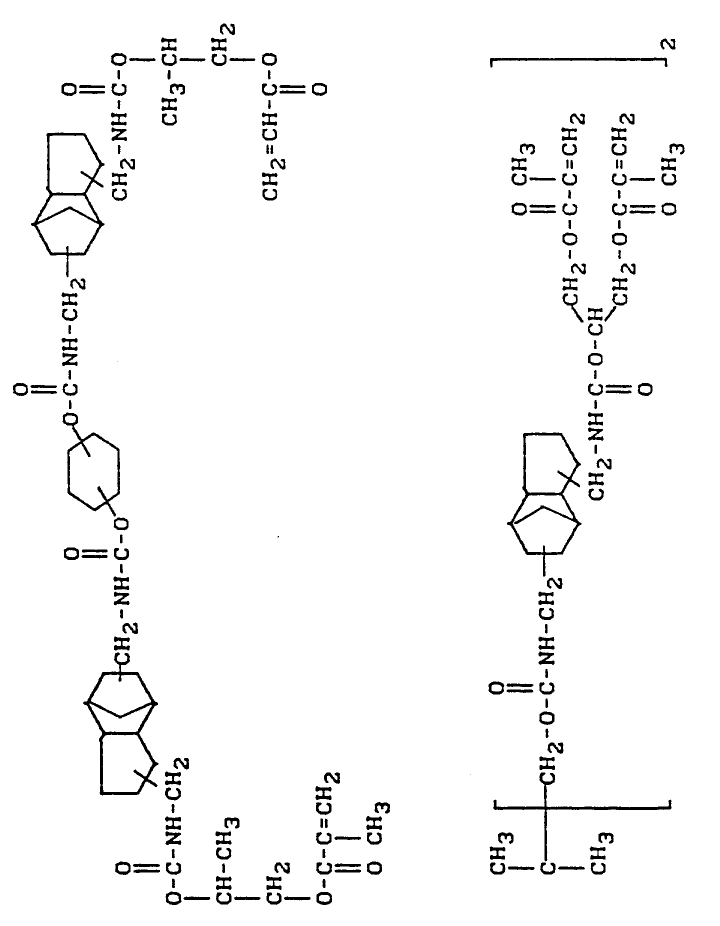

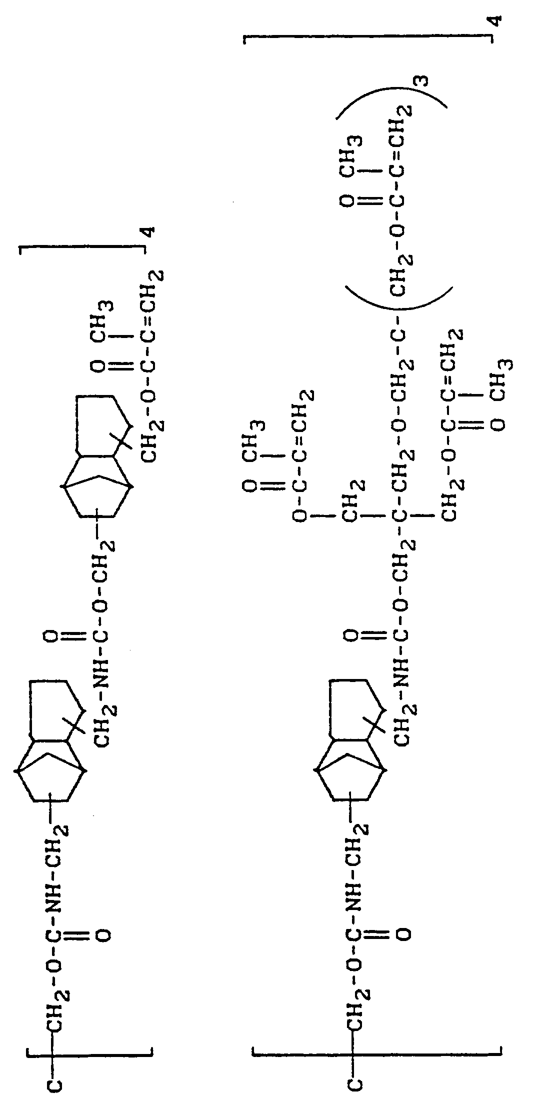

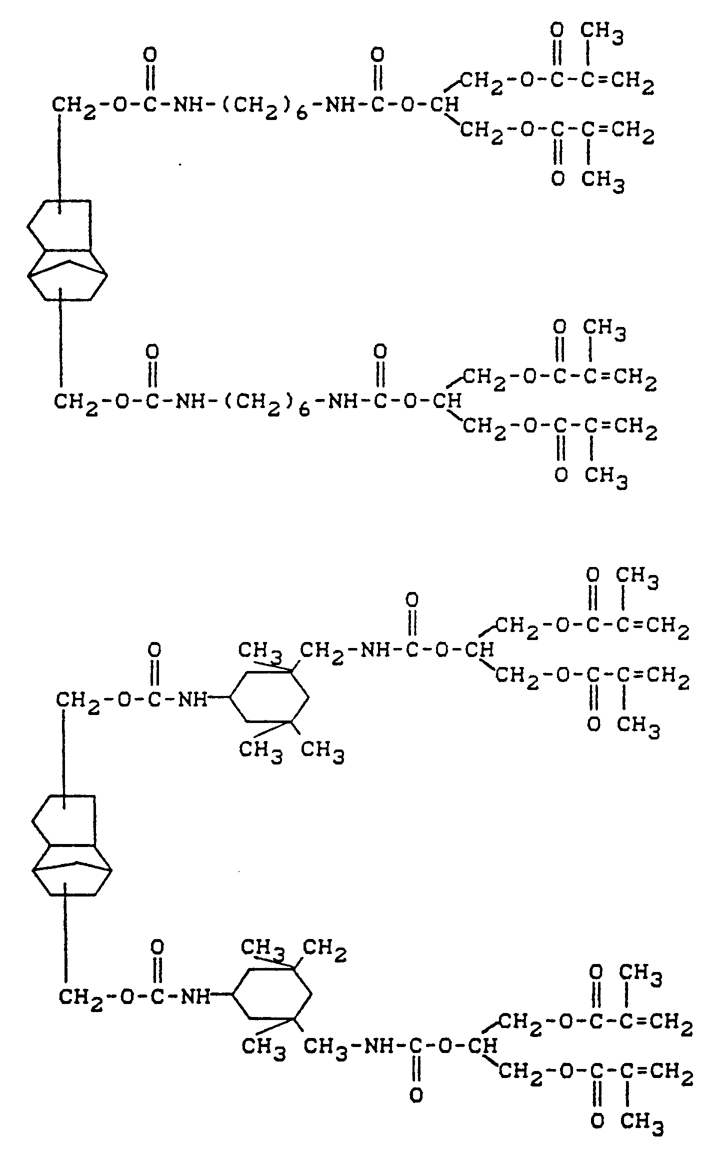

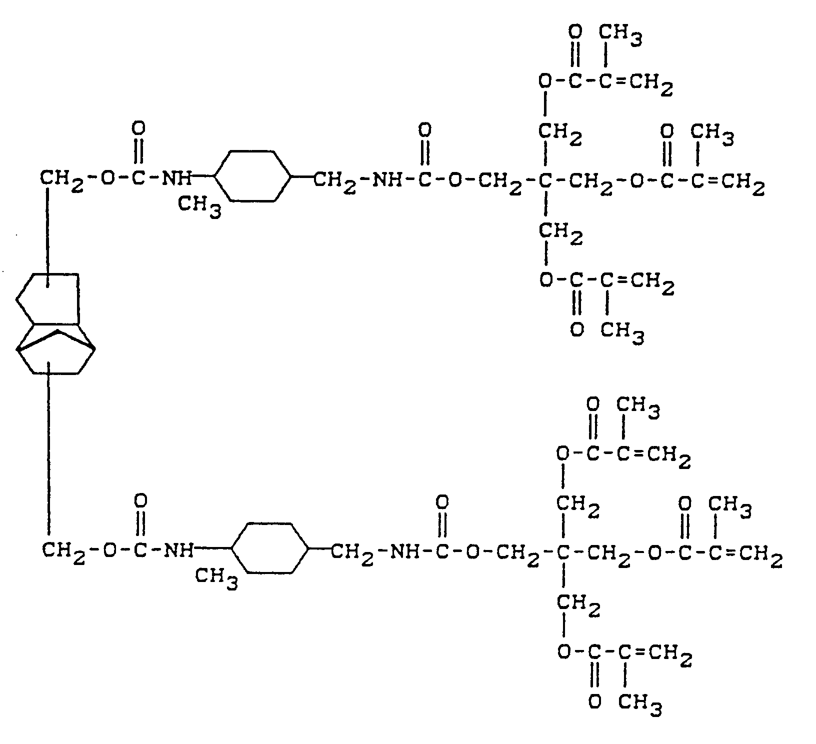

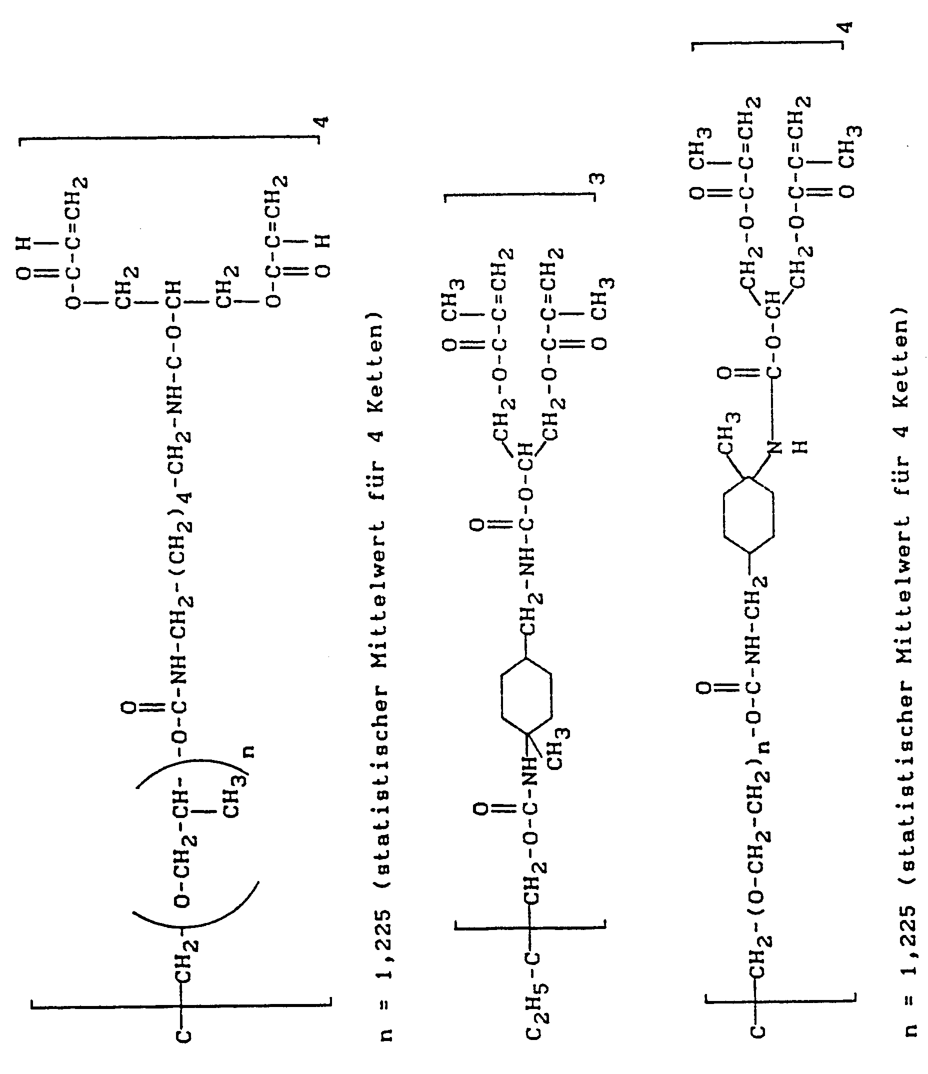

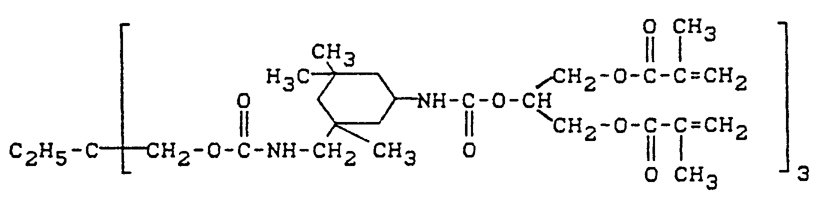

Besonders bevorzugt als Monomer wird das sogenannte Bis-GMA der Formel

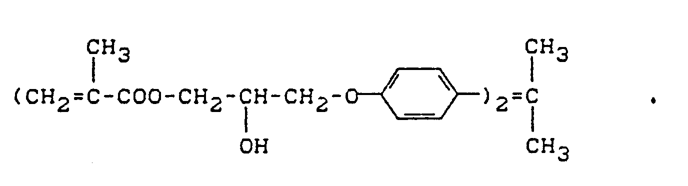

Selbstverständlich ist es möglich, Mischungen der verschiedenen (Meth)-Acrylsäureester, die Vernetzungen ausbilden können, einzusetzen. Beispielsweise seien Mischungen aus 20 bis 70 Gew.-Teilen Bis-GMA und 30 bis 80 Gew.-Teilen Triethylenglykoldimethacrylat genannt.

### Komponente (c)

(Meth)-Acrylsäureester, die keine Vernetzungen ausbilden können, sind im allgemeinen monofunktionelle (Meth)-Acrylate. Beispielsweise seien C₁-C₁₂-, vorzugsweise C₁-C₄-Alkylmethacrylate wie Methylmethacrylat, Ethylmethacrylat, n-Propylmethacrylat, i-Propylmethacrylat, n-Butylmethacrylat, tert.-Butylmethacrylat, Hydroxyalkylmethacrylate wie 2-Hydroxyethylmethacrylat, 2-Hydroxypropylmethacrylat, Diethylenglykolmonomethacrylat und Triethylenglykolmonomethacrylat und Alkoxyalkylmethacrylate wie 2-Methoxyethylmethacrylat, 3-Methoxybutylmethacrylat und Ethyltriglykolmethacrylat genannt.

Bevorzugte (Meth)-Acrylsäureester, die keine Vernetzungen ausbilden können, sind Methylmethacrylat, Ethylmethacrylat, Hydroxyethylmethacrylat.

Im allgemeinen setzt man für die erfindungsgemäßen Dentalwerkstoffe die Komponenten (b) und (c) in Mischung ein. Diese Mischungen haben bevorzugt eine Viskosität im Bereich von 50 bis 5000 mPa.s, vorzugsweise im Bereich von 100 bis 2000 mPa.s (jeweils bei 20°C).

### Komponente (d)

Die anorganischen Füllstoffe können z.B. aus kugelig, annähernd kugelig oder durch Mahlung oder direkt durch die Herstellung (z.B. in flammhydrolytischen Verfahren hergestellte) erzeugten regelmäßig oder unregelmäßig geformten Partikeln bestehen. Die anorganischen Füllstoffe können mit einer monomodalen, bimodalen oder polymodalen Teilchengrößenverteilung eingesetzt werden. Die Füllstoffe haben im allgemeinen ein Maximum in der Verteilungskurve im Bereich von 0,005 bis 5 µm. Geeignete anorganische Füllstoffe sind beispielsweise Gläser in Form von Kugeln oder in Form von durch Mahlung gewonnenen unregelmäßig geformten Partikeln mit mittleren Teilchengrößen im Bereich von 0,5 bis 5 µm. Des weiteren geeignet sind flammhydrolytisch gewonnene Oxide des Siliciums und Aluminiums mit Teilchengrößen im Bereich von 5 bis 500 nm. Die BET-Oberfläche der anorganischen Füllstoffe beträgt 20 bis 600 m²/g, vorzugsweise 50 bis 300 m²/g. Die anorganischen Füllstoffe gelangen in mit Haftvermittlern behandelter Form zum Einsatz.

Als Haftvermittler eignen sich beispielsweise Silan- und Titanatverbindungen, wie Trimethylchlorsilan, Hexamethyldisiloxan, 3-Aminopropyltrimethoxysilan, Butyltitanat und Isopropyltitanat. Besonders gut geeignet sind Haftvermittler mit polymerisierbaren Gruppen, wie Vinyltrimethylsiloxan, Allyltrimethoxysilan und γ-Methacryloyloxypropyltrimethoxysilan.

Die Nachbehandlung der Füllstoffe ist an sich bekannt. Die Behandlung mit Silanverbindungen erfolgt vorteilhafterweise in einem nicht-wäßrigen Lösungsmittel wie Toluol, Methylcyclohexan, Aceton, Tetrahydrofuran oder Methylethylketon, wobei die Reaktion mit Säuren oder Aminen katalysiert wird.

Die geeignete Menge an Haftvermittlern richtet sich nach der Größe der Oberfläche des anorganischen Füllstoffs. Im allgemeinen ist eine Menge von 0,5 bis 5 % gut geeignet.

### Komponente (e)

Die erfindungsgemäßen Dentalwerkstoffe können an sich bekannte Additive enthalten. Als Additive seien Starterzusätze, Stabilisatoren, Pigmente, Farbstoffe, Lichtschutzmittel, Fluoreszenzmittel oder Weichmacher genannt.

Als Starterzusätze für die Einleitung der Polymerisation können an sich bekannte Startersysteme (Literatur Houben Weyl, Methoden der organischen Chemie Band E20, S. 15 ff., Georg Thieme Verlag, Stuttgart 1987) verwendet werden. Es handelt sich um Radikale, Anionen oder Kationen liefernde Systeme, die eine radikalische, anionische oder kationische Polymerisation auslösen können. Im Falle von Radikale lieferndem System sind Peroxide oder aliphatische Azoverbindungen besonders geignet, beispielsweise Benzoylperoxid, Lauroylperoxid oder Azoisobuttersäurenitril; die Systeme werden üblicherweise in Mengen von 0,1 bis 5 Gew.-% eingesetzt. Während die Härtung bei erhöhter Temperatur allein durch Peroxide oder andere Radikalstarter durchgeführt werden kann, ist zur Härtung bei Raumtemperatur im allgemeinen ein Zusatz von Beschleunigern, vorzugsweise aromatischen Aminen, zweckmäßig. Geeignete Beschleuniger sind z.B. N,N-substituierte Toluidine und Xylidine, wie N,N-Dimethyl-p-toluidin oder N,N-Bis-(2-Hydroxyethyl)-xylidin. Die Aushärtung kann im allgemeinen durch Zusatz von 0,5 bis 3 Gew.-% der genannten Amine erreicht werden.

Es ist jedoch auch möglich, Dentalwerkstoffe herzustellen, die unter Einwirkung von Licht, beispielsweise UV-Licht, sichtbarem Licht oder Laserlicht, polymerisieren. In diesen Fällen setzt man Fotopolymerisationsinitiatoren und Beschleuniger ein.

Fotopolymerisationsinitiatoren sind an sich bekannt (Literatur Houben Weyl, Methoden der organischen Chemie, Band E20, S. 80 ff, Georg Thieme Verlag Stuttgart 1987).

Vorzugsweise handelt es sich dabei um Carbonylverbindungen, wie Benzoin und dessen Derivate, insbesondere Benzoinmethylether, Benzoyl-und Benzylderivate, beispielsweise Benzil und andere Dicarbonylverbindungen wie Diacetyl, 2,3-Pentandion oder Metallcarbonyle wie Pentacarbonylmangan, Chinone wie 9,10-Phenanthrenchinon und Campherchinon oder deren Derivate.

Dar Anteil an solchen Fotopolymerisationsinitiatoren beträgt vorzugsweise etwa 0,01 bis etwa 5 Gew.-% der Gesamtzusammensetzung.

Die mit Licht härtbaren fotopolymerisierbaren Massen enthalten vorzugsweise noch Substanzen, die in Gegenwart von Fotopolymerisationsinitiatoren die Polymerisationsreaktion beschleunigen. Bekannte Beschleuniger sind beispielsweise aromatische Amine wie p-Toluidin und Dimethyl-p-toluidin, Trialkylamine wie Trihexylamin, Polyamine wie N,N,N',N'-Tetraalkylalkylendiamin, Barbitursäure und Dialkylbarbitursäure und Sulfimide.

Die Beschleuniger werden im allgemeinen in einer Menge von 0,01 bis etwa 5 Gew.-% der Gesamtmischung eingesetzt.

Es ist auch möglich, den erfindungsgemäßen Dentalwerkstoffen UV-Stabilisatoren zuzusetzen, um das Nachdunkeln während des Alterns zu vermeiden.

Ein besonders geeigneter UV-Stabilisator ist 2-Hydroxy-4-methoxybenzophenon. Ein weiteres bevorzugtes Material ist 2-(2-Hydroxy-5-methylphenyl)-benzotriazol. Beispielsweise seien noch Hydrochinon, p-Benzochinon und p-Butylhydroxytoluol genannt.

Zur Einstellung einer möglichst naturgetreuen Farbe können die erfindungsgemäßen Dentalwerkstoffe auch an sich bekannte Pigmente und Farbstoffe enthalten.

Die Polymerisation wird im allgemeinen unter den oben genannten Bedingungen ausgeführt.

Die erfindungsgemäßen Dentalwerkstoffe können unter Formgebung zu künstlichen Zähnen und Zahnersatzteilen, wie Kronen, Brücken, Inlays und Onlays, zu Zahnfüllungen und Zahnlacken verarbeitet werden, Die polymerisierten erfindungsgemäßen Dentalwerkstoffe zeichnen sich durch eine günstige Eigenschaftskombination aus. Sie besitzen eine hohe Härte, eine hohe Steifigkeit und eine hohe Abrasionsbeständigkeit bei guter Zähigkeit. Sie können leicht eingefärbt und der natürlichen Zahnfarbe angepaßt werden.

### Beispiel 1

### Herstellung eines Polymethacrylsäureesters aus Ethylenglykoldimethacrylat

In einem 3-Liter-Glasreaktor, der mit Blattrührer, Rückflußkühler, Innenthermometer, Gaseinlaß-und Gasauslaßrohr ausgerüstet ist, werden 1800 g Butanon-2, 200 g Ethylenglykoldimethacrylat und 2 g Dibenzoylperoxid eingewogen. Unter Rühren mit 300 UpM und unter Stickstoffspülung wird 2 Stunden am Rückfluß erhitzt, Es entsteht eine gut rührbare Suspension. Aus dieser lassen sich durch Sprühtrocknung 190 g feines Pulver gewinnen. Die mittlere Teilchengröße (gemessen mit Laserkorrelationsspektroskopie) beträgt 700 nm, der Gelgehalt 98,4 % und der Quellungsgrad (gemessen in Tetrahydrofuran) 310 %.

### Beispiel 2

### Herstellung eines Polymethacrylsäureesters aus Glycerindimethacrylat

Nach der in Beispiel 1 angegebenen Arbeitsweise werden 500 g Glycerindimethacrylat und 5 g Dibenzoylperoxid in 2000 g Butanon-2 umgesetzt. Man erhält 475 g Pulver mit einer Teilchengröße von 350 nm, einem Gelgehalt von 97,3 % und einem Quellungsgrad von 280 %.

### Beispiel 3

### Polymerisierbare Masse

25 g Polymerisat aus Beispiel 1, 65 g Bis-GMA, 35 g Triethylenglykoldimethacrylat, 40 g mikrofeines Siliciumdioxid (mit einer BET-Oberfläche von 130 m²/g, silanisiert mit 5 % γ-Methacryloyloxypropyltrimethoxysilan) und 0,4 g Dibenzoylperoxid werden in einem Laborkneter 30 Minuten verknetet. Die erhaltene Mase wird 5 Stunden bei 35°C gelagert. Man erhält eine nicht klebende, transparente, teigartige Paste.

### Beispiel 4

### Polymerisierbare Masse

20 g Polymerisat aus Beispiel 1, 65 g Bis-GMA, 35 g Triethylenglykoldimethacrylat, 50 g mikrofeines Siliciumdioxid (mit einer BET-Oberfläche von 90 m²/g, silanisiert mit 3 % γ-Methacryloyloxypropyltrimethoxysilan) und 0,4 g Dibenzoylperoxid werden in einem Laborkneter 30 Minuten verknetet. Die erhaltene Masse wird 5 Stunden bei 35°C gelagert. Man erhält eine nicht klebende, transparente, teigartige Paste.

### Beispiel 5

Die Massen aus den Beispielen 3 und 4 sowie 3 Vergleichsmaterialien wurden bei 140°C und 200 bar in 10 Min. zu einer Prüfplatte polymerisiert. Es wurden Werte für die Biegefestigkeit nach DIN 13 922 und den Biege-E-Modul nach DIN 13 922 sowie die Eindringtiefe nach Wallace ermittelt.

Die Wallacemethode dient zur Bestimmung der Eindringhärte an Kunststoffen. Ein Vickers-Diamant wird unter einer Vorlast von 1 p auf die Oberfläche aufgebracht und anschließend 60 sec lang mit einer Hauptlast von 100 p beansprucht. Als Maß für den Eindringwiderstand wird die Eindringtiefe des Diamanten unter Hauptlast gemessen. Im Gegensatz zu den Vickers- oder Brinellhärtemessungen, bei denen die Prüfkraft auf die Dimension des bleibenden Eindrucks bezogen wird, erfaßt man mit der Wallacemethode die elastische und die bleibende Verformung des Kunststoffes.

| | Biegefestigkeit N/mm² | E-Modul N/mm² | HW µm |
|---|---|---|---|
| Beispiel 3 | 138 ± 11 | 5200 ± 160 | 15,2 |
| Beispiel 4 | 146 ± 6 | 5400 ± 120 | 14,8 |
| übliches PMMA-System | 112 ± 9 | 2800 ± 100 | 22,8 ± 1,5 |
| US 4 396 377 Beispiel 1 | 118 ± 10 | 3050 ± 80 | 20,3 ± 1,1 |
| EP-A-0 346 707 Beispiel 3 | 140 ± 7 | 3800 ± 150 | 16,2 ± 0,9 |

## Patentansprüche

1. Dentalwerkstoffe aus
a) 5 bis 30 Gew.-Teilen eines organischen Füllstoffs, bestehend aus polymeren vernetzten (Meth)-Acrylaten mit einer Teilchengröße im Bereich von 0,001 bis 100 µm, einem Quellungsgrad von 50 bis 2000 Gew.-% und einem Vernetzungsgrad von 50 bis 100 Gew.-%, jeweils bezogen auf das Polymer,
b) 40 bis 80 Gew.-Teilen(Meth)-Acrylate, die Vernetzungen ausbilden können,
c) 0 bis 40 Gew.-Teilen(Meth)-Acrylate, die keine Vernetzungen ausbilden können,
d) 10 bis 40 Gew.-Teilen eines silanisierten anorganischen Füllstoffs mit einer mittleren Teilchengröße von 0,005 bis 5 µm und
e) 0,1 bis 10 Gew.-%, bezogen auf die Gesamtzusammensetzung, eines oder mehrer Additive.

2. Dentalwerkstoffe nach Anspruch 1, dadurch gekennzeichnet, daß der organische Füllstoff eine BET-Oberfläche von 20 bis 600 m²/g aufweist.

3. Dentalwerkstoffe nach den Ansprüchen 1 und 2, dadurch gekennzeichnet, daß der anorganische Füllstoff eine BET-Oberfläche von 50 bis 250 m²/g aufweist.

4. Dentalwerkstoffe nach den Ansprüchen 1 bis 3, dadurch gekennzeichnet, daß die Mischung der (Meth)-Acrylsäureester, die Vernetzungen und keine Vernetzungen ausbilden können, eine Viskosität im Bereich von 50 bis 5000 mPa.s aufweist.

5. Verfahren zur Herstellung von Dentalwerkstoffen, dadurch gekennzeichnet, daß man eine Mischung aus
a) 5 bis 30 Gew.-Teile eines organischen Füllstoffes, bestehend aus polymeren vernetzten (Meth)-Acrylate mit einer Teilchengröße im Bereich von 0,001 bis 100 µm, einem Quellungsgrad von 50 bis 2000 Gew.-% und einem Vernetzungsgrad von 50 bis 100 Gew.-%, jeweils bezogen auf das Polymer,
b) 40 bis 80 Gew.-Teilen (Meth)-Acrylate, die Vernetzungen ausbilden können, und
c) 0 bis 40 Gew.-Teilen (Meth)-Acrylate, die keine Vernetzungen ausbilden können und
d) 10 bis 40 Gew.-Teile eines silanisierten anorganischen Füllstoffs mit einer mittleren Teilchengröße von 0,005 bis 5 µm,
polymerisiert.

6. Verwendung von Dentalwerkstoffen nach den Ansprüchen 1 bis 4 zur Herstellung von künstlichen Zähnen, Zahnersatzteilen, Zahnfüllungen und Zahnlacken.

## Claims

1. Dental materials comprising
a) from 5 to 30 parts by weight of an organic filler, consisting of polymeric crosslinked (meth)acrylates having a particle size in the range from 0.001 to 100 µm, a degree of swelling of from 50 to 2000% by weight and a degree of crosslinking of from 50 to 100% by weight, based in each case on the polymer,
b) from 40 to 80 parts by weight of (meth)acrylates which can form crosslinkages,
c) from 0 to 40 parts by weight of (meth)acrylates which cannot form crosslinkages,
d) from 10 to 40 parts by weight of a silanized inorganic filler having a mean particle size of from 0.005 to 5 µm and
e) from 0.1 to 10% by weight, based on the total composition, of one or more additives.

2. Dental materials according to Claim 1, characterized in that the organic filler has a BET surface area of from 20 to 600 m²/g.

3. Dental materials according to Claims 1 and 2, characterized in that the inorganic filler has a BET surface area of from 50 to 250 m²/g.

4. Dental materials according to any of Claims 1 to 3, characterized in that the mixture of the (meth)acrylates which can form crosslinkages and those which cannot form crosslinkages has a viscosity in the range from 50 to 5000 mPa.s.

5. Process for the preparation of dental materials, characterized in that a mixture of
a) from 5 to 30 parts by weight of an organic filler, consisting of polymeric crosslinked (meth)acrylates having a particle size in the range from 0.001 to 100 µm, a degree of swelling of from 50 to 2000% by weight and a degree of crosslinking of from 50 to 100% by weight, based in each case on the polymer,
b) from 40 to 80 parts by weight of (meth)acrylates which can form crosslinkages,
c) from 0 to 40 parts by weight of (meth)acrylates which cannot form crosslinkages and
d) from 10 to 40 parts by weight of a silanized inorganic filler having a mean particle size of from 0.005 to 5 µm.
is polymerized.

6. The use of dental materials according to any of Claims 1 to 4 for the production of artificial teeth, dental prostheses, dental fillings and dental lacquers.

## Revendications

1. Matériaux dentaires comprenant
a) de 5 à 30 parties en poids d'une charge organique, constituée de (méth)acrylates réticulés polymères présentant une dimension de particules comprise dans la gamme de 0,001 à 100 µm, un degré de gonflement de 50 à 2000 % en poids et un degré de réticulation de 50 à 100 % en poids, respectivement par rapport au polymère,
b) de 40 à 80 parties en poids de (méth)acrylates qui peuvent former des réticulations,
c) de 0 à 40 parties en poids de (méth)acrylates qui ne peuvent pas former des réticulations,
d) de 10 à 40 parties en poids d'une charge inorganique silanée présentant une dimension moyenne de particules comprise entre 0,005 et 5 µm et
e) de 0,1 à 10 % en poids, par rapport à la composition totale, d'un ou plusieurs additifs.

2. Matériaux dentaires selon la revendication 1, caractérisés en ce que la charge organique présente une surface BET de 20 à 600 m²/g.

3. Matériaux dentaires selon les revendications 1 et 2, caractérisés en ce que la charge inorganique présente une surface BET de 50 à 250 m²/g.

4. Matériaux dentaires selon les revendications 1 à 3, caractérisés en ce que le mélange des esters d'acide (méth)acrylique, qui peuvent former des réticulations et qui ne peuvent pas en former, présente une viscosité comprise dans la gamme de 50 à 5000 mPa.s.

5. Procédé de préparation de matériaux dentaires, caractérisé en ce qu'on polymérise un mélange comprenant :
a) de 5 à 30 parties en poids d'une charge organique, constituée de (méth)acrylates réticulés polymères présentant une dimension de particules comprise dans la gamme de 0,001 à 100 µm, un degré de gonflement de 50 à 2000 % en poids et un degré de réticulation de 50 à 100 % en poids, respectivement par rapport au polymère,
b) de 40 à 80 parties en poids de (méth)acrylates qui peuvent former des réticulations,
c) de 0 à 40 parties en poids de (méth)acrylates qui ne peuvent pas former des réticulations et
d) de 10 à 40 parties en poids d'une charge inorganique silanée présentant une dimension moyenne de particules comprise entre 0,005 et 5 µm.

6. Utilisation de matériaux dentaires selon les revendications 1 à 4 pour la préparation de dents artificielles, d'éléments dentaires de remplacement, de plombages dentaires et de vernis dentaires.
